# EUROPÄISCHE PATENTSCHRIFT

(11) **EP 2 747 803 B1**
(45) Veröffentlichungstag und Bekanntmachung des Hinweises auf die Patenterteilung: **16.11.2016**
(21) Anmeldenummer: 12769307.5
(22) Anmeldetag: 27.09.2012
(51) Int. Cl.: A61M 1/00, A61M 1/06

(54) **VAKUUMPUMPE**
VACUUM PUMP
POMPE A VIDE

(30) Priorität: 04.10.2011 CH 16272011
(43) Veröffentlichungstag der Anmeldung: 02.07.2014
(73) Patentinhaber: Medela Holding AG, 6340 Baar (CH)
(72) Erfinder: FELBER, Armin, 6003 Luzern (CH)
(74) Vertreter: Clerc, Natalia
(86) Internationale Anmeldenummer: PCT/CH2012/000222
(87) Internationale Veröffentlichungsnummer: WO 2013/049944

(56) Entgegenhaltungen:
- WO-A1-2006/032156
- WO-A1-2011/035447
- US-A1- 2003 116 702
- US-A1- 2004 265 149
- US-A1- 2007 060 873
- US-A1- 2007 078 383
- US-A1- 2008 255 503
- US-A1- 2010 179 472

## Beschreibung

### TECHNISCHES GEBIET

Die vorliegende Erfindung betrifft eine Vakuumpumpe gemäss Oberbegriff des Patentanspruchs 1.

### STAND DER TECHNIK

Vakuumpumpen dienen der Erzeugung von Unterdruck. Sie lassen sich beispielsweise als Brustpumpen zum Abpumpen von Muttermilch und als Drainagepumpen, insbesondere im Bereich der Thorax- oder Wunddrainage, einsetzen. Membranvakuumpumpen lassen sich relativ klein und kostengünstig ausbilden.

WO 2011/035447 offenbart eine motorbetriebene Brustpumpe zum Abpumpen von Muttermilch mit einer Vakuummembran, welche gleichzeitig zur Erzeugung oder Weiterleitung des Vakuums, zur Medientrennung zwischen Luft und Milch und als Transportmittel für die abgesaugte Milch dient. Die Vakuummembran ist entweder direkt über einen Motor und mechanische Kraftübertragungsmittel angetrieben oder über eine Vakuumleitung mit einem vakuumerzeugenden Pumpaggregat verbunden.

WO 2008/057218 offenbart ebenfalls eine Vakuummembran einer Brustpumpe. Diese Vakuummembran ist über eine Druckleitung mit einem Pumpaggregat verbunden. Sie weist eine angeformte Ventilklappe auf.

Insbesondere bei Drainagepumpen ist eine Messung des erzeugten bzw. des beim Benützer angelegten Vakuums bekannt. Üblicherweise wird innerhalb der Vakuumpumpe in einer

Vakuumleitung oder in einer externen Saug- oder Vakuumleitung der Unterdruck mit Sensoren bekannter Art gemessen. Beispielsweise werden hierfür piezoelektrische Sensoren eingesetzt. Bei Brustpumpen ist die Messung des erzeugten Unterdrucks bisher nicht üblich.

Im Stand der Technik ist eine Vielfalt von Drucksensoren bekannt. So offenbart US 2003/0116702 einen optischen Drucksensor mit einem Gehäuse, einer darin gehaltenen Membran mit Lichtblocker, einer Photodiode, einem Photodetektor und Prismen, welche Licht von der Photodiode zum Photodetektor leiten. Die Position der Membran und somit die Lage des Lichtblockers im Strahlweg zwischen Photodiode und Photodetektor ist vom Druckverhältnis im Gehäuse abhängig.

WO 03/034014 offenbart einen Drucksensor zur Bestimmung eines Fluiddrucks, wobei der Druck über eine Membran an einen Sensor übertragen wird.

Auch WO 2011/027117 betrifft einen Drucksensor, welcher eine Membran zur Übertragung des Drucks verwendet.

US2007/0060873 beschreibt eine weitere Membranpumpe mit Drucksensor.

### DARSTELLUNG DER ERFINDUNG

Es ist eine Aufgabe der Erfindung, eine verbesserte Vakuumpumpe mit einem Drucksensor zu schaffen, welche sich insbesondere als Brustpumpe zum Abpumpen von menschlicher Muttermilch einsetzen lässt.

Diese Aufgabe löst eine Vakuumpumpe mit den Merkmalen des Patentanspruchs 1.

Die erfindungsgemässe Vakuumpumpe zur Erzeugung eines Unterdrucks weist eine Pumpkammer mit einem Eingang und einem Ausgang auf, wobei der Ausgang mit einem Ventil ausgestattet ist. Vorzugsweise ist der Ausgang mit dem Ventil, insbesondere einem Rückschlagventil versehen. Erfindungsgemäss ist die Pumpkammer mit einem Drucksensor versehen bzw. in der Pumpkammer ist ein Drucksensor angeordnet.

Durch die Anordnung des Drucksensors an oder in der Vakuumkammer, hier auch Pumpkammer genannt, lässt sich die Vakuumpumpe klein und kompakt ausbilden. Es wird keine zusätzliche Leitung zu einem Drucksensor benötigt. Durch die Anordnung des Sensorelements an oder in der Vakuumkammer wird der Unterdruck vor Ort gemessen, es sind keine Verluste und Undichtheiten zu berücksichtigen. Die Bestimmung des Unterdrucks ist somit relativ genau.

Der gemessene Vakuumwert lässt sich zur Steuerung der Pumpe einsetzen, auf einem Display der Pumpe für den Benützer anzeigen oder anderweitig verwenden.

Der Drucksensor umfasst eine Sensormembran, welche in Abhängigkeit des in der Pumpkammer herrschenden Drucks ausgelenkt wird und so einem Detektor den herrschenden Druck bzw. die Grösse einer Druckänderung übermittelt. Die Sensormembran bildet somit das Sensorelement.

In einer bevorzugten Ausführungsform ist an der Sensormembran eine Fahne angeordnet, deren Lage relativ zur Pumpkammer detektierbar ist. Die Fahne und die restliche Sensormembran sind vorzugsweise gemeinsam einstückig ausgebildet, wobei die Fahne vorzugsweise im Vergleich zur restlichen Sensormembran steifer ist. Die Fahne bildet somit ein Übertragungselement, welches die Information bezüglich der Auslenkung der Sensormembran, d.h. die Druckveränderung, weiter leitet.

Die Detektion der Auslenkung der Sensormembran, insbesondere die Lageveränderung der abstehenden Fahne, kann mit bekannten Mitteln, beispielsweise kapazitiv oder induktiv, erfolgen. In einer bevorzugten Ausführungsform erfolgt die Detektion der Lage der Membran durch ein optisches Detektionsmittel.

Vorzugsweise umfasst das optische Detektionsmittel einen Lichtemitter und einen Lichtdetektor, wobei die Fahne durch Auslenkung der Sensormembran in einen Lichtpfad zwischen Lichtemitter und Lichtdetektor bewegbar ist. Die detektierte Lichtmenge ist abhängig davon, wie weit die Fahne in den Lichtpfad hineinragt, und ergibt somit nach entsprechender Umrechnung die Auslenkung der Sensormembran und dadurch den in der Pumpkammer herrschenden Druck.

Dieser Sensor ist in einer Membranvakuumpumpe eingesetzt, in welcher eine Vakuummembran eine Wand der Pumpkammer bildet, wobei das Volumen der Pumpkammer durch Bewegung der Vakuummembran verkleinert und vergrössert wird und somit der Unterdruck in der Pumpkammer zyklisch erzeugt wird.

In einer bevorzugten Ausführungsform sind Vakuummembran und Sensormembran gemeinsam einstückig ausgebildet. Vorzugsweise bildet die Sensormembran in diesem Fall eine einseitige Verlängerung der Vakuummembran. Sie ist vorzugsweise in einem Randbereich der Vakuummembran angeordnet.

In einer anderen bevorzugten Ausführungsform sind die Vakuummembran und die Sensormembran zwei voneinander getrennt ausgebildete Teile. Vorzugsweise sind in diesem Fall beide rund ausgebildet.

Die Sensormembran und die Vakuummembran sind vorzugsweise aus Silikon oder einem thermoplastischen Elastomer (TPE) gefertigt. Die Sensormembran kann dünner oder dicker ausgebildet sein als die Vakuummembran. Vorzugsweise weist sie dieselbe Dicke, insbesondere 0.8 mm auf.

Insbesondere die Vakuummembran ist vorzugsweise tellerförmig ausgebildet mit kreisförmigen Vertiefungen und Erhebungen.

Die Pumpkammer weist einen ersten Kammerbereich und einen mit diesem ersten Kammerbereich über eine Durchgangsöffnung verbundenen zweiten Kammerbereich auf. Beide Kammerbereiche weisen dasselbe Druckniveau auf. Der erste Kammerbereich dient als Pumpbereich, der zweite als Sensorbereich. Die Vakuummembran überdeckt den ersten Kammerbereich und die Sensormembran den zweiten Kammerbereich. Im ersten Kammerbereich ist der Eingang und im zweiten Kammerbereich der Ausgang angeordnet.

Die Durchgangsöffnung ist im Vergleich zum ersten Kammerbereich verjüngt ausgebildet und besteht vorzugsweise aus einem Verbindungskanal zwischen dem ersten und dem zweiten Kammerbereich. Der zweite Kammerbereich kann denselben Durchmesser aufweisen wie der Verbindungskanal, oder er kann breiter bzw. mit einem grösseren Volumen versehen sein. Das Volumen des Verbindungskanals und/oder der Durchmesser der Durchgangsöffnung bleibt auch bei sich bewegender Sensormembran und Vakuummembran konstant. Die Volumina des ersten und des zweiten Kammerbereichs ändern sich bei sich bewegenden Membranen. Der erste Kammerbereich ist vorzugsweise wesentlich grösser ausgebildet als der zweite Kammerbereich.

In einer bevorzugten Ausführungsform weist die Vakuummembran einen Mittelpunkt auf, wobei dieser Mittelpunkt mit einem Antriebselement zur Betätigung der Vakuummembran verbindbar ist. In diesem Mittelpunkt ist vorzugsweise ein Verbindungskopf zur Verbindung mit dem Antriebsaggregat angeordnet.

Die erfindungsgemässe Vakuumpumpe lässt sich insbesondere als Brustpumpe zum Abpumpen von Muttermilch oder als Drainagepumpe, insbesondere für die Wund- oder Thoraxdrainage einsetzen. Die Vakuummembran kann direkt über einen Motor und eine mechanische Kraftübertragungseinheit, beispielsweise eine Pleuelstange, angetrieben sein. Sie kann jedoch auch manuell betrieben oder über eine Vakuumleitung mit einem motor- oder manuellbetriebenen vakuumerzeugenden Pumpaggregat verbunden sein.

Insbesondere lässt sich die brusthaubenseitige Pumpkammer der medientrennenden Membran gemäss der WO 2011/035447 mit einem derartigen Drucksensor ausstatten.

Weitere Ausführungsformen sind in den abhängigen Ansprüchen angegeben.

### KURZE BESCHREIBUNG DER ZEICHNUNGEN

Bevorzugte Ausführungsformen der Erfindung sind im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung einer erfindungsgemässen Vakuumpumpe in der Verwendung als Brustpumpe;
- Figur 2: die Vakuumpumpe gemäss Figur 1 in teilweiser Explosionsdarstellung;
- Figur 3: einen vergrösserten Ausschnitt der Vakuumpumpe gemäss Figur 2;
- Figur 4: einen Teil der Vakuumpumpe Figur 2 in einer weiteren vergrösserten Explosionsdarstellung mit einer leicht anders ausgebildeten Sensoreinheit;
- Figur 5: einen Längsschnitt durch die Pumpkammer der Vakuumpumpe gemäss Figur 1 in einer ersten Stellung der Membran;
- Figur 6: einen Längsschnitt durch die Pumpkammer der Vakuumpumpe gemäss Figur 1 in einer zweiten Stellung der Membran;
- Figur 7: eine perspektivische Explosionsdarstellung einer erfindungsgemässen Vakuumpumpe gemäss einer zweiten Ausführungsform und in der Verwendung als Brustpumpe und
- Figur 8: eine zweite perspektivische Explosionsdarstellung der Vakuumpumpe gemäss Figur 7.

### BESCHREIBUNG BEVORZUGTER AUSFÜHRUNGSFORMEN

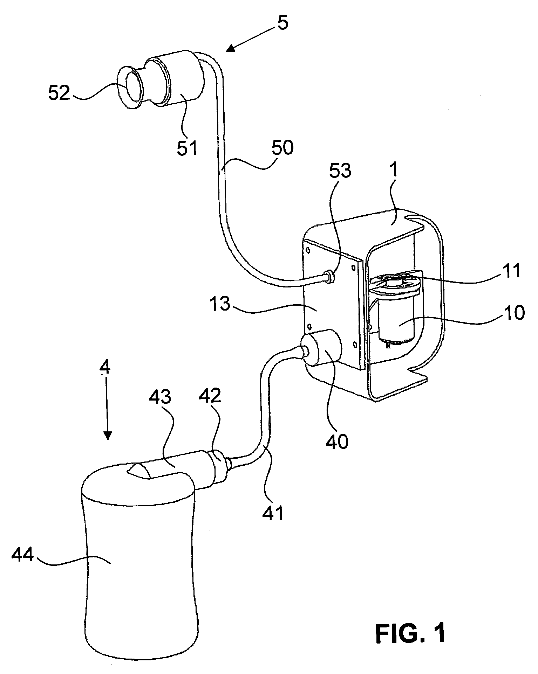

In Figur 1 ist ein erstes Ausführungsbeispiel der erfindungsgemässen Vakuumpumpe in Form einer Brustpumpe zum Abpumpen von menschlicher Muttermilch dargestellt. Sie weist ein Gehäuse 1 einer Vakuumpumpe mit einem Elektromotor 10 auf. Eine Brusthaubeneinheit 5 umfasst eine Brusthaube 51 mit einem auf eine Mutterbrust dichtend aufsetzbaren Brusthaubentrichter 52 sowie eine erste Leitung 50. Diese erste Leitung 50 ist über ein erstes Kopplungsteil 53 mit dem Gehäuse 1 der Vakuumpumpe lösbar verbindbar. Eine Milchsammeleinheit 4 weist einen Milchsammelbehälter 44 mit einem Verbindungsstutzen 43 sowie eine zweite Leitung 41 auf. Diese zweite Leitung 41 ist mit einem zweiten Kopplungsteil 40 versehen, mit welchem sie mit dem Gehäuse 1 der Vakuumpumpe lösbar verbindbar ist. Am gegenüberliegenden Ende der zweiten Leitung 41 ist ein drittes Kopplungsteil 42 vorhanden, welches in den Verbindungsstutzen 43 einsteckbar oder über ihn stülpbar ist.

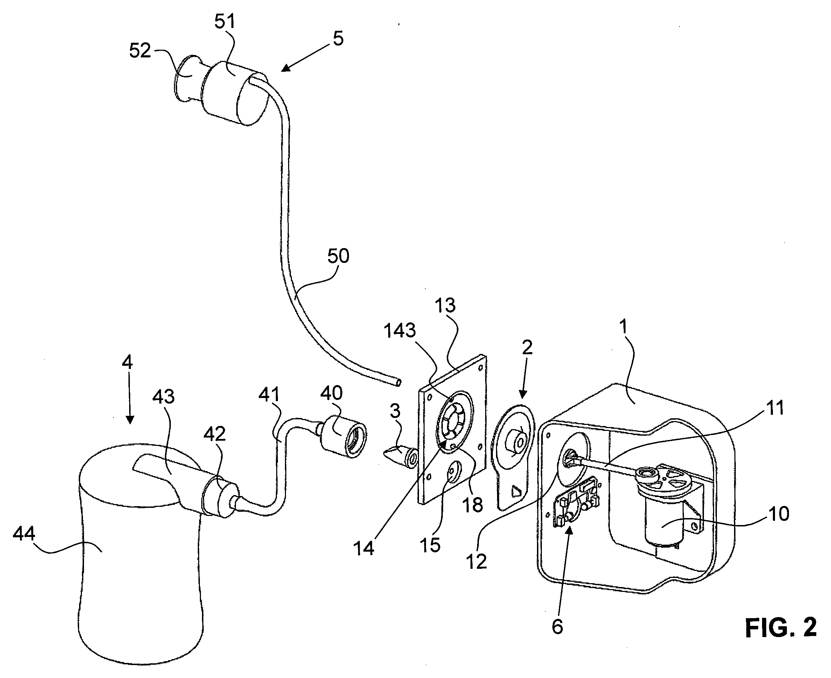

Wie in Figur 2 erkennbar ist, ist ein Deckel 13 auf einer Aussenwand des Gehäuses 1 befestigt. Vorzugsweise ist er lösbar befestigt, beispielsweise verschraubt. In diesem Deckel 13 ist, dem Gehäuse 1 zugewandt, eine Pumpkammer ausgebildet. Sie besteht aus zwei Kammerbereichen 14, 15. Beide Kammerbereiche 14, 15 sind im Wesentlichen kreisförmig ausgebildet und bilden je eine Vertiefung im Deckel 13. Der erste Kammerbereich 14 weist einen wesentlich grösseren Durchmesser auf als der zweite Kammerbereich 15. Die zwei Kammerbereiche 14, 15 sind über einen Verbindungskanal 18 miteinander verbunden. Der Verbindungskanal 18 ist bis auf seine zwei in die Kammerbereiche 14, 15 führenden Mündungsöffnungen vollständig geschlossen. Er weist üblicherweise keine Ventile auf.

Im ersten Kammerbereich 14 ist eine Eingangsöffnung 143 vorhanden, mit welcher das erste Kopplungsteil 53 der Brusthaubeneinheit 5 verbindbar ist. Hier ist es eingesteckt. Im zweiten Kammerbereich 15 ist eine Ausgangsöffnung 150 vorhanden, welche gegen die Aussenseite des Deckels 13 mit einem Ventil 3 versehen ist. Vorzugsweise ist diese Ausgangsöffnung 150 mittig im kreisförmigen Kammerbereich 15 angeordnet. Das Ventil ist vorzugsweise ein Rückschlagventil, insbesondere ein Schnabelventil. Andere Ventilarten und -typen lassen sich auch verwenden.

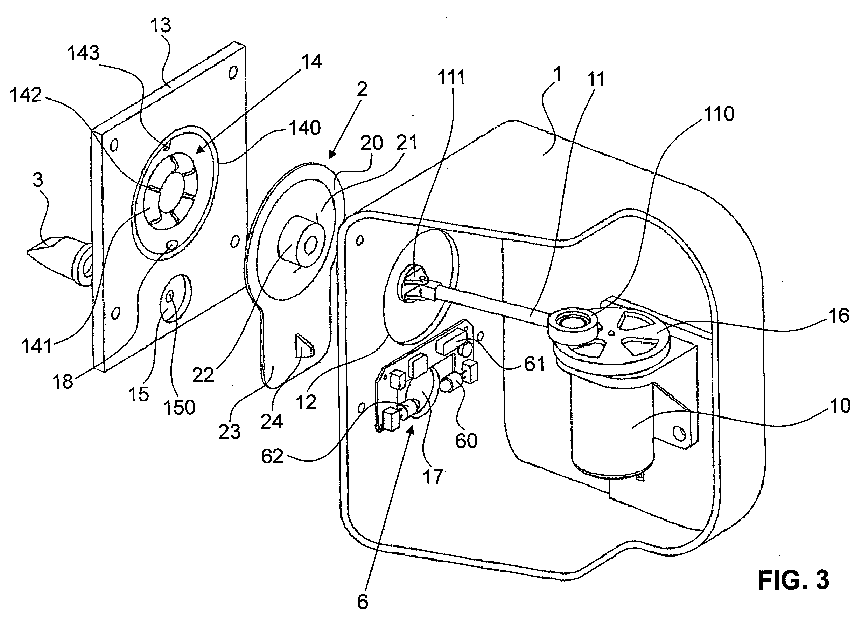

Der erste Kammerbereich 14 weist einen kreisförmigen Dichtrand 140 auf. Beabstandet zu diesem Dichtrand 140 ist ein geschlossener Ring 141 mit einer innen liegenden, vorzugsweise planen Grundfläche 144 vorhanden. Der Ring ist von Radialrillen 142 durchsetzt, welche die Grundfläche 144 mit der Aussenseite des Rings 141 verbinden. Die Eingangsöffnung 143 und das diesseitige Ende des Verbindungskanals 18 befinden sich zwischen Dichtrand 140 und Ring 141, wobei sie vorzugsweise einander diametral gegenüberliegen. Dies ist in Figur 3 gut erkennbar.

Im bestimmungsgemässen Gebrauch der Brustpumpe ist die Eingangsöffnung 143 vorzugsweise über dem Verbindungskanal 18 angeordnet bzw. der erste Kammerbereich 14 oberhalb des zweiten Kammerbereichs 15.

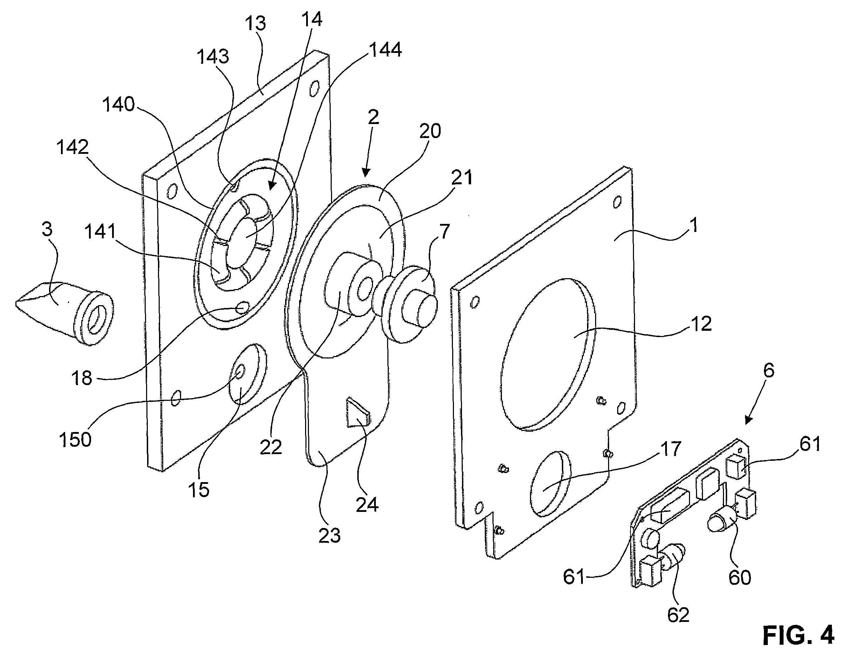

Zwischen Deckel 13 und der Aussenwand des Gehäuses 1 ist eine Membran 2 angeordnet. Diese Membran 2 ist vorzugsweise aus Silikon oder einem thermoplastischen Elastomer (TPE) gefertigt. Die Membran 2 weist einen kreisförmigen Membrankörper 21 mit einem annähernd vollständig umlaufenden Rand 20 auf. Der Membrankörper 21 mit seinem Rand 20 ist vorzugsweise tellerförmig ausgebildet. Er weist vorzugsweise Erhebungen und Vertiefungen auf. Mittig im Membrankörper 21 ist ein Verbindungskopf 22 einstückig angeformt oder befestigt. Dieser Verbindungskopf 22 ist über ein Kopplungsteil 7 (siehe Figur 4) mit einer Kraftübertragungsstange 11 des Antriebs lösbar oder fest verbunden.

Wie in Figur 3 gut erkennbar ist, weist der Antrieb den genannten Elektromotor 10 auf, welcher mit einer Drehscheibe 16 versehen ist. Auf der Drehscheibe 16 ist ein Pleuelkopf 110 der Kraftübertragungsstange 11, beispielsweise einer Pleuelstange, angeordnet. Ein dem Pleuelkopf 110 entgegen gesetztes Ende der Kraftübertragungsstange 11 ist als Verbindungselement 111 ausgebildet. Dieses Verbindungselement 111 ist über das Kopplungsteil 7 mit der Membran 2 verbunden. Hierfür weist das Gehäuse 1 eine erste Durchgangsöffnung 12 auf. Somit wird eine Rotationsbewegung des Motors 10 über die Kraftübertragungsstange 11 in eine lineare Bewegung der Membran 2, genauer des Membrankörpers 21, übertragen.

Der Membrankörper 21 überdeckt den ersten Kammerbereich 14, insbesondere den Ring 141 und die von ihm umschlossene Kavität. Durch die lineare Bewegung der Membran 2 entlang der Längsachse des Verbindungskopfes 22 lässt sich im Innern der Kavität und somit im ersten Kammerbereich 14 ein Unterdruck erzeugen. Membrankörper 21 und Rand 20 bilden somit eine Vakuummembran zur Erzeugung eines Unterdrucks. Der erste Kammerbereich 14 bildet einen Pumpbereich zur Erzeugung des Vakuums.

Die Membran 2 weist ferner eine einseitige Verlängerung in Form eines Lappens 23 auf. Der Lappen 23 ist hier im Wesentlichen rechteckig und plan ausgebildet. An diesem Lappen 23 ist eine Nase oder Fahne 24 angeformt, welche dem Lappen 23 senkrecht in Richtung Gehäuse 1 vorsteht. Die Fahne 24 ist vorzugsweise steifer ausgebildet als der Lappen 23 und als die restliche Membran 20, 21. Einzig der Verbindungskopf 22 weist vorzugsweise eine gleiche oder grössere Steifheit auf.

Der Lappen 23 überdeckt den zweiten Kammerbereich 15, wobei die Fahne 24 im montierten Zustand vorzugsweise über die Ausgangsöffnung 150 zu liegen kommt. Zumindest liegt die Fahne 24 im zweiten Kammerbereich 15. Auch der Bereich des Lappens 23 ist dichtend zwischen Deckel 13 und Aussenseite der Gehäusewand 1 gehalten, so dass die Pumpkammer, gebildet durch die zwei Kammerbereiche 14 und 15, gegenüber dem Gehäuse 1 abgedichtet ist. Die Aussenseite der Gehäusewand 1 ist vorzugsweise plan ausgebildet. Der Bereich des Lappens 23, welcher über dem zweiten Kammerbereich 15 liegt, bildet eine Sensormembran. Der zweite Kammerbereich 15 bildet einen Sensorbereich, wie nachfolgend dargelegt wird.

Im Bereich der Fahne 24 weist das Gehäuse 1 eine zweite Durchgangsöffnung 17 auf, durch welche die Fahne 24 hindurchragen kann. Beidseitig dieser zweiten Durchgangsöffnung 17, auf der Innenseite des Gehäuse 1, ist eine optische Sensoreinheit 6 angeordnet. Sie weist einen Lichtemitter 60, z. B. eine Photodiode, und einen Lichtdetektor 62, z.B. eine Photodiode, auf. Lichtemitter 60 und Lichtdetektor 62 sind vorzugsweise gemeinsam mit einer Steuerungs- und Auswerteelektronik 61 auf einer gemeinsamen Leiterplatine angeordnet. Lichtemitter 60 und Lichtdetektor 62 sind einander diametral gegenüberliegend am Rand der zweiten Durchgangsöffnung 17 angeordnet. Lichtemitter 60 und Lichtdetektor 62 definieren einen Lichtpfad oder Strahlengang, welcher senkrecht zur Fahne 24 verläuft und von dieser durchsetzbar ist.

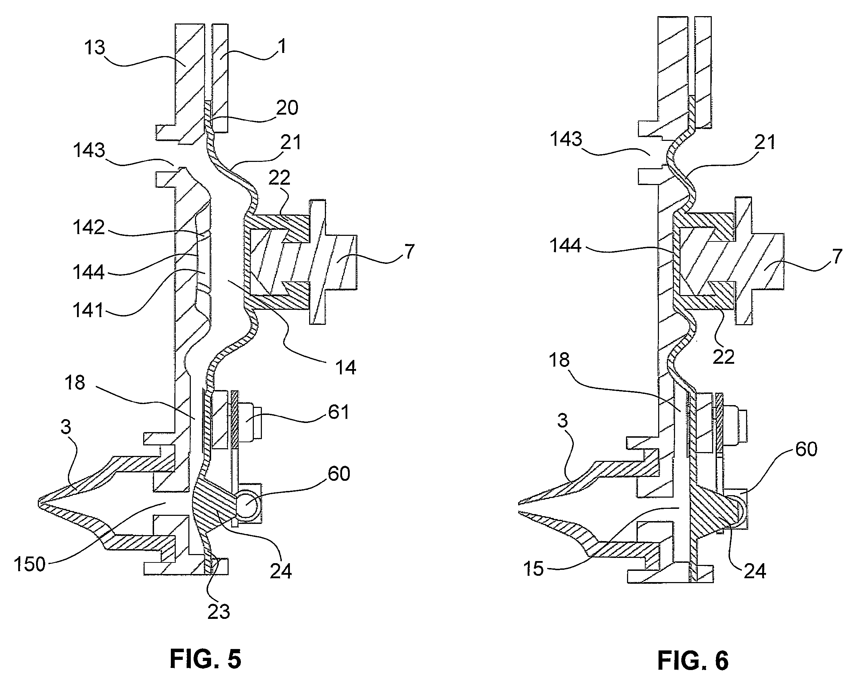

In den Figuren 5 und 6 ist nun die Bewegung der Membran 2 erkennbar. In beiden Figuren ist erkennbar, wie der Rand 20 und die Peripherie des Lappens 23 dichtend zwischen dem Gehäuse 1 und dem Deckel 13 eingespannt sind.

In Figur 5 wird die Membran 2, konkret der Membrankörper 21, über das Kopplungsteil 7 und den Antrieb 1 zum Gehäuse 1 hin gezogen. Das Volumen des ersten Kammerbereichs 14 wird vergrössert. Luft aus der ersten Leitung 50 der Brusthaubeneinheit 5 wird in die Kammer gesogen. Das Rückschlagventil 3 ist geschlossen. Durch den im ersten Kammerbereich 14 entstehende Unterdruck wird auch die Sensormembran, das heisst der über dem zweiten Kammerbereich 15 liegende Teil des Lappens 23, in den zweiten Kammerbereich 15 hinein gezogen. Dadurch bewegt sich der Lappen 23 zur Ausgangsöffnung 150 hin und somit weg vom Lichtemitter 60 und Lichtdetektor 62. Der Lappen 23 bewegt sich somit mindestens teilweise aus dem Lichtpfad heraus.

In Figur 6 ist die Membran 2, konkret wiederum der Membrankörper 21, zur Grundfläche 144 hingepresst. Vorzugweise liegt der Membrankörper 21 auf dieser Grundfläche 144 auf. Die Verbindung mit der ersten Leitung 50 der Brusthaubeneinheit 5 ist vorzugsweise unterbrochen, indem die Membran 2 die Eingangsöffnung 143 verschliesst. Das Ventil 3 ist geöffnet. Die Sensormembran 23 ist entspannt und plan und die Fahne 24 ragt in den Lichtpfad hinein und überdeckt somit mindestens teilweise den Lichtemitter 60. Dies entspricht für die Messung des Vakuums einer Nullstellung. Je nach erzeugtem Vakuum bzw. Unterdruck wird, wie in Figur 5 erkennbar, die Sensormembran mit der Fahne 24 unterschiedlich weit aus dem Strahlengang gezogen, so dass in Abhängigkeit des erzeugten Vakuums eine bestimmte Lichtmenge vom Lichtemitter zum gegenüberliegenden Lichtdetektor gelangen kann. Diese Lichtmenge wird von der Steuer- und Auswerteelektronik 61 bestimmt und einem Vakuumwert zugeordnet. Der gemessene Vakuumwert entspricht genau dem Vakuumwert der Pumpkammer, da der erste Kammerbereich 14 und der zweite Kammerbereich 15 aufgrund des Verbindungskanals 18 stets auf einem gemeinsamen Druckniveau liegen. Die Position der Fahne 24 im Lichtpfad kann auch ohne Zuordnung eines konkreten Vakuumwertes an eine Steuereinheit der Pumpe übermittelt und zur Steuerung der Pumpe verwendet werden. Des Weiteren kann anstelle einer optischen Sensoreinheit 6 auch ein anderes Mittel zur Detektion der Lage der Fahne 24 eingesetzt werden.

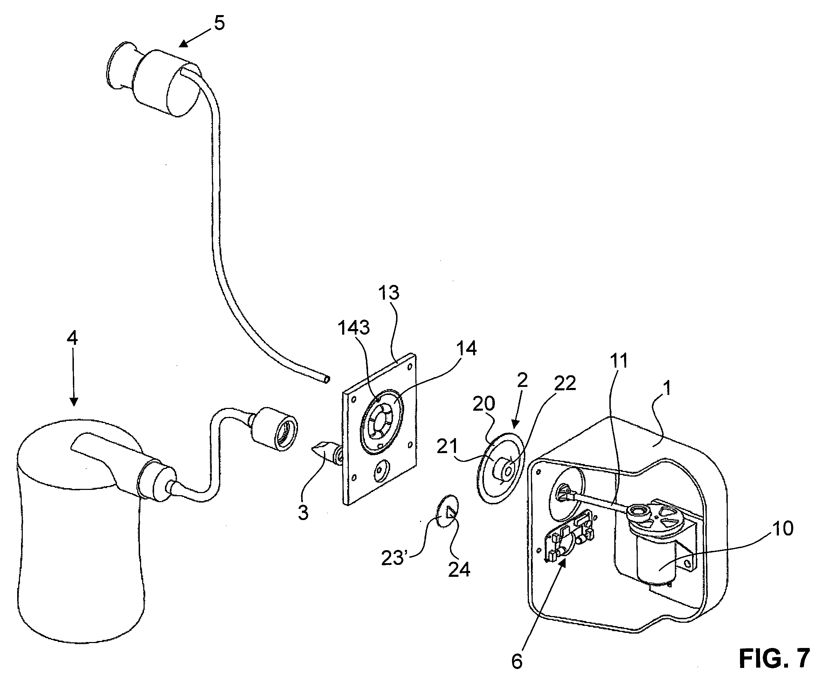

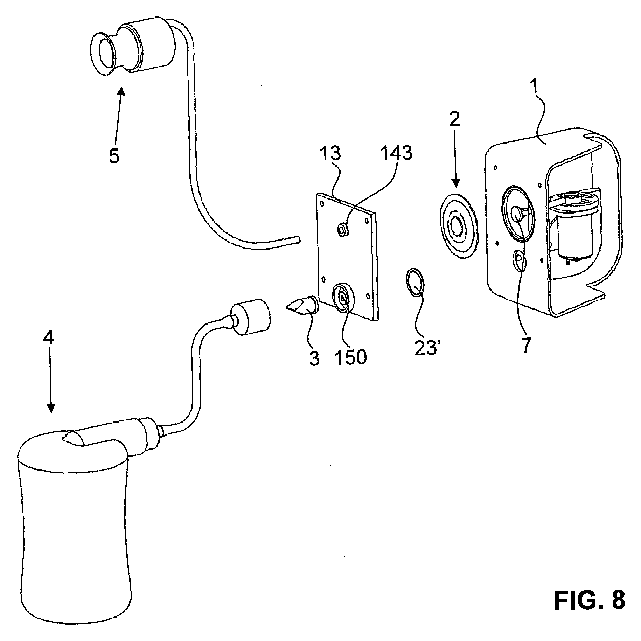

In den Figuren 7 und 8 ist ein zweites Ausführungsbeispiel dargestellt. Auch hier wird die erfindungsgemässe Vakuumpumpe als Brustpumpe zum Abpumpen von menschlicher Muttermilch eingesetzt. Gleiche Teile sind analog zum ersten Ausführungsbeispiel mit denselben Bezugsziffern bezeichnet und werden hier nicht mehr im Detail beschrieben. Im Gegensatz zum ersten Beispiel ist die Membran 2 jedoch nicht mehr einstückig dargestellt. Vakuummembran und Sensormembran sind zwei voneinander getrennt ausgebildete Membranen. Die Vakuummembran ist rund ausgebildet und wird durch den Membrankörper 21 und den Rand 20 gebildet. Die Sensormembran 23' ist hier ebenfalls rund und vorzugsweise plan ausgebildet und weist die vorstehende Fahne 24 auf. Sie kann einen umlaufenden Dichtrand aufweisen.

Wie bereits in WO 2011/035447 beschrieben, wird in beiden oben genannten Beispielen die abgepumpte Milch durch die Vakuum- oder Pumpkammer gepumpt. Die Membran 2 dient somit als vakuumerzeugende Membran, zum Transport der Milch und als Medientrennung zwischen der Luft im Gehäuse der Pumpe und der Milch. Diese Membran 2 lässt sich auch gemäss den weiteren Ausführungsbeispielen der WO'447 mit einem vakuumerzeugenden Pumpaggregat koppeln, so dass sie durch einen an sie über eine Unterdruckleitung applizierten zyklischen Unterdruck bewegt wird. In diesem Fall erzeugt sie nach wie vor einen Unterdruck in der Pumpkammer 14, wobei sich dieser Unterdruck proportional zum angelegtem zyklischen Unterdruck des Vakuumaggregats verhält. Die übrigen genannten Funktionen bleiben dadurch erhalten.

Die erfindungsgemässe Vakuumpumpe verfügt über einen relativ genauen und kostengünstigen Vakuumsensor. Vorteilhaft ist zudem, dass der Sensor, da integriert in die Pumpkammer, kaum störungsanfällig ist und einen geringen Platzbedarf aufweist.

**BEZUGSZEICHENLISTE**

| | | | |
|---|---|---|---|
| 1 | Gehäuse | 24 | Fahne |
| 10 | Elektromotor | | |
| 11 | Kraftübertragungsstange | 3 | Ventil |
| 110 | Pleuelkopf | | |
| 111 | Verbindungselement | 4 | Milchsammeleinheit |
| 12 | erste Durchgangsöffnung | 40 | zweites Kopplungsteil |
| 13 | Deckel | 41 | zweite Leitung |
| 14 | erster Kammerbereich | 42 | drittes Kopplungsteil |
| 140 | Dichtrand | 43 | Verbindungsstutzen |
| 141 | Ring | 44 | Milchsammelbehälter |
| 142 | Radialrille | | |
| 143 | Eingangsöffnung | 5 | Brusthaubeneinheit |
| 144 | Grundfläche | 50 | erste Leitung |
| 15 | zweiter Kammerbereich | 51 | Brusthaube |
| 150 | Ausgangsöffnung | 52 | Brusthaubentrichter |
| 16 | Drehscheibe | 53 | erstes Kopplungsteil |
| 17 | zweite Durchgangsöffnung | | |
| 18 | Verbindungskanal | 6 | optische Sensoreinheit |
| | | 60 | Lichtemitter |
| 2 | Membran | 61 | Steuer- und |
| 20 | Rand | | Auswerteelektronik |
| 21 | Membrankörper | 62 | Lichtdetektor |
| 22 | Verbindungskopf | | |
| 23 | Lappen | 7 | Kopplungsteil |
| 23' | Sensormembran | | |

## Patentansprüche

1. Vakuumpumpe zur Erzeugung eines Unterdrucks, wobei die Vakuumpumpe eine Pumpkammer (14, 15) aufweist mit einem Eingang (143) und einem Ausgang (150), wobei der Ausgang (150) mit einem Ventil (3) ausgestattet ist und wobei die Pumpkammer (14, 15) mit einem Drucksensor (23, 23', 24) versehen ist und der Drucksensor eine Sensormembran (23, 23') umfasst, wobei die Vakuumpumpe eine Vakuummembran (20, 21) zur Erzeugung des Unterdrucks in der Pumpkammer (14, 15) aufweist und die Pumpkammer einen ersten Kammerbereich (14) und einen mit diesem ersten Kammerbereich (14) über eine Durchgangsöffnung (18) verbundenen zweiten Kammerbereich (15) aufweist, wobei im ersten Kammerbereich (14) der Eingang (143) und im zweiten Kammerbereich (15) der Ausgang (15) angeordnet ist, **dadurch gekennzeichnet, dass** die Vakuummembran (20, 21) den ersten Kammerbereich (14) überdeckt und die Sensormembran (23, 23') den zweiten Kammerbereich (15).

2. Vakuumpumpe nach Anspruch 1, wobei an der Sensormembran (23, 23') eine Fahne (24) angeordnet ist, deren Lage relativ zur Pumpkammer (14, 15) detektierbar ist.

3. Vakuumpumpe nach Anspruch 2, wobei ein optisches Detektionsmittel (6) vorhanden ist, um die Lage der Fahne (24) zu detektieren.

4. Vakuumpumpe nach Anspruch 3, wobei das optische Detektionsmittel (6) einen Lichtemitter (60) und einen Lichtdetektor (62) umfasst und wobei die Fahne (24) durch Auslenkung der Sensormembran (23, 23') in einen Lichtpfad zwischen Lichtemitter (60) und Lichtdetektor (62) bewegbar ist.

5. Vakuumpumpe nach Anspruch 1, wobei die Vakuummembran (20, 21) und die Sensormembran (23, 23') gemeinsam einstückig ausgebildet sind.

6. Vakuumpumpe nach Anspruch 5, wobei die Sensormembran (23) eine einseitige Verlängerung der Vakuummembran (20, 21) ist.

7. Vakuumpumpe nach Anspruch 1, wobei die Vakuummembran (20, 21) und die Sensormembran (23') zwei voneinander getrennt ausgebildete Teile sind.

8. Vakuumpumpe nach Anspruch 1, wobei die Vakuummembran (20, 21) im Wesentlichen einen runden Querschnitt aufweist.

9. Vakuumpumpe nach Anspruch 8, wobei die Vakuummembran (20, 21) einen Mittelpunkt aufweist, wobei dieser Mittelpunkt mit einen Antriebselement (10, 11) zur Betätigung der Vakuummembran (20, 21) verbindbar ist.

10. Vakuumpumpe nach Anspruch 9, wobei im Mittelpunkt ein Verbindungskopf (22) zur Verbindung mit dem Antriebselement (10, 11) angeordnet ist.

## Claims

1. Vacuum pump for generating an underpressure, wherein the vacuum pump has a pump chamber (14, 15) with an inlet (143) and an outlet (150), wherein the outlet (150) is equipped with a valve (3) and wherein the pump chamber (14, 15) is provided with a pressure sensor (23, 23', 24) and wherein the pressure sensor comprises a sensor diaphragm (23, 23'), wherein the vacuum pump comprises a vacuum diaphragm (20, 21) for generating the underpressure in the pump chamber (14, 15) and wherein the pump chamber has a first chamber area (14) and a second chamber area (15), which is connected to this first chamber area (14) via a through-opening (18), wherein the inlet (143) is arranged in the first chamber area (14), and the outlet (150) is arranged in the second chamber area (15), **characterized in that** the vacuum diaphragm (20, 21) covers the first chamber area (14), and the sensor diaphragm (23, 23') covers the second chamber area (15).

2. Vacuum pump according to Claim 1, wherein a vane (24) is arranged on the sensor diaphragm (23, 23'), and the position of the vane (24) relative to the pump chamber (14, 15) is detectable.

3. Vacuum pump according to Claim 2, wherein an optical detection means (6) is present in order to detect the position of the vane (24).

4. Vacuum pump according to Claim 3, wherein the optical detection means (6) comprises a light emitter (60) and a light detector (62), and wherein the vane (24) is movable, by deflection of the sensor diaphragm (23, 23'), into a light path between light emitter (60) and light detector (62).

5. Vacuum pump according to Claim 1, wherein the vacuum diaphragm (20, 21) and the sensor diaphragm (23, 23') are formed together in one piece.

6. Vacuum pump according to Claim 5, wherein the sensor diaphragm (23) is an extension of one side of the vacuum diaphragm (20, 21).

7. Vacuum pump according to Claim 1, wherein the vacuum diaphragm (20, 21) and the sensor diaphragm (23') are two parts formed separately from each other.

8. Vacuum pump according to Claim 1, wherein the vacuum diaphragm (20, 21) has substantially a round cross section.

9. Vacuum pump according to Claim 8, wherein the vacuum diaphragm (20, 21) has a central point, and wherein this central point is connectable to a drive element (10, 11) for the actuation of the vacuum diaphragm (20, 21).

10. Vacuum pump according to Claim 9, wherein a connection head (22) for connection to the drive element (10, 11) is arranged at the central point.

## Revendications

1. Pompe à vide pour la production d'une dépression, dans laquelle la pompe à vide présente une chambre de pompe (14, 15) avec une entrée (143) et une sortie (150), dans laquelle la sortie (150) est équipée d'une soupape (3) et dans laquelle la chambre de pompe (14, 15) est munie d'un capteur de pression (23, 23', 24) et le capteur de pression comprend une membrane de capteur (23, 23'), dans laquelle la pompe à vide présente une membrane à vide (20, 21) pour la production d'une dépression dans la chambre de pompe (14, 15) et la chambre de pompe présente une première région de chambre (14) et une deuxième région de chambre (15) reliée à cette première région de chambre (14) par une ouverture de passage (18), dans laquelle l'entrée (143) est disposée dans la première région de chambre (14) et la sortie (15) est disposé dans la deuxième région de chambre (15), **caractérisée en ce que** la membrane à vide (20, 21) recouvre la première région de chambre (14) et la membrane de capteur (23, 23') recouvre la deuxième région de chambre (15).

2. Pompe à vide selon la revendication 1, dans laquelle un tenon (24), dont la position par rapport à la chambre de pompe (14, 15) peut être détectée, est disposé sur la membrane de capteur (23, 23').

3. Pompe à vide selon la revendication 2, dans laquelle il se trouve un moyen de détection optique (6), destiné à détecter la position du tenon (24).

4. Pompe à vide selon la revendication 3, dans laquelle le moyen de détection optique (6) comprend un émetteur de lumière (60) et un détecteur de lumière (62) et dans laquelle le tenon (24) est déplaçable, par déplacement de la membrane de capteur (23, 23'), dans un chemin de lumière entre l'émetteur de lumière (60) et le détecteur de lumière (62).

5. Pompe à vide selon la revendication 1, dans laquelle la membrane à vide (20, 21) et la membrane de capteur (23, 23') forment ensemble une seule pièce.

6. Pompe à vide selon la revendication 5, dans laquelle la membrane de capteur (23) est un prolongement unilatéral de la membrane à vide (20, 21).

7. Pompe à vide selon la revendication 1, dans laquelle la membrane à vide (20, 21) et la membrane de capteur (23') sont deux pièces formées séparément l'une de l'autre.

8. Pompe à vide selon la revendication 1, dans laquelle la membrane à vide (20, 21) présente essentiellement une section transversale ronde.

9. Pompe à vide selon la revendication 8, dans laquelle la membrane à vide (20, 21) présente un point central, dans laquelle ce point central peut être relié à un élément d'entraînement (10, 11) pour l'actionnement de la membrane à vide (20, 21).

10. Pompe à vide selon la revendication 9, dans laquelle une tête de liaison (22) est disposée au point central pour la liaison avec l'élément d'entraînement (10, 11).
